# EUROPEAN PATENT APPLICATION

(11) **EP 1 997 826 A2**
(43) Date of publication of application: **03.12.2008**
(21) Application number: 08009793.4
(22) Date of filing: 29.05.2008
(51) Int. Cl.: C07K 5/10, C07K 7/02, A61K 38/10, A61K 38/08, A61K 38/07

(54) **Synthetic ligands for immunoglobulins and pharmaceutical compositions containing them**

(30) Priority: 01.06.2007 IT MI20071119
(71) Applicant: Tecnogen S.p.A., 81015 Piana di Monte Verna (Caserta) (IT)
(72) Inventor: Verdoliva, Antonio, 80143 Napoli (IT); Rossi, Maria, 81100 Caserta (IT); Manfredi, Vincenzo, 80053 Castellammare di Stabia (NA) (IT); Ruvo, Menotti, 83058 Trevico (AV) (IT); Colombo, Maurizio, 80046 S. Giorgio Cremasco (NA) (IT)
(74) Representative: Colombo, Stefano Paolo

(57) **Abstract**

A peptide comprising the sequence of formula:

X₁-Arg-Thr-Tyr- (S)

where:
X₁ is the residue of an amino acid, of an amino acid derivative, of a carboxylic acid, or of some other derivative capable of binding covalently to the amino group of arginine, comprising at least one aromatic group.

Pharmaceutical composition comprising the aforesaid peptide.

Use of the aforesaid peptide for production of a pharmaceutical composition for the treatment of allergic diseases.

## Description

### Field of the invention

The present invention relates to novel synthetic peptides that are able to bind non-covalently to immunoglobulins, and pharmaceutical compositions containing them.

The immunoglobulins find wide application in therapeutics, where they are used as agents capable of binding to biological molecules involved in therapeutically important physiological processes. Considering their importance, it also follows that their production, and especially their purification, assume particular industrial significance.

The immunoglobulins can be obtained from animal sera or from the cultivation of suitable cell lines, and their purification is normally carried out by conventional methods involving precipitation with inorganic salts or organic solvents, such as ammonium sulphate and ethanol, chromatographic techniques, such as ion exchange, gel filtration, and electrophoretic techniques.

However, these methods cannot usually be applied easily on a large scale for industrial applications and always involve a compromise between yield and product purity. At present the most widely used industrial method for the purification of antibodies from biological fluids is affinity chromatography and, in particular, chromatography on columns prepared by immobilizing Protein A.

Protein A, extracted from Staphylococcus aureus, is able to bind specifically and with high affinity to the constant portion of the immunoglobulins, permitting rapid and selective purification of antibodies (Siodahl, J., 1977. Eur. J. Biochem. 78, 471-490; Fuglistaller, P., 1989. J. Immunol. Methods 124, 171).

However, wide-scale application of Protein A, obtained from microorganisms or from genetically modified bacteria, has many limitations.

It is in fact produced by complex and expensive methods that normally require numerous analytical tests for detecting the possible presence of contaminants that may impair the quality of the purified product (viruses, pyrogens, DNA etc.). Furthermore, Protein A is not very stable in denaturing conditions or in the presence of agents used for removing biological contaminants such as viruses or fragments of nucleic acids.

From the therapeutic standpoint, it is known that inhibition of interaction between the immunoglobulins and the corresponding antigen or cell-bound receptors represents an important therapeutic approach for many pathologies.

For example, in multiple myeloma, experimental data have shown that immunotherapy based on the administration of soluble Fc receptors reduces the growth of the tumour cells and the secretion of immunoglobulins (Hoover et al. 1995, J. Clin. Invest. 95, 241-247).

Moreover, the sera of AIDS patients contain antibodies that increase viral infectivity by interacting with the respective cellular receptors (Homsy et al., 1989, Science 244, 1357-1360) and, consequently, the administration of molecules that are able to interfere with lg/receptor interaction represents an important therapeutic approach for viral infection by HIV.

Also in diseases of inflammatory origin, such as rheumatoid arthritis, in which the event that leads to the pathological condition is represented by Ig/receptor interaction (Fearon and Wong, 1983, Ann. Rev. Immunol. 1, 243), a treatment based on compounds that are able to block this interaction can offer notable therapeutic benefits.

Finally, in the allergic response the interaction of IgEs with their receptors on the surface of mast cells and basophilic leukocytes triggers, on successive contact of the immunoglobulins with the antigen, a series of metabolic processes culminating in degranulation and the release of the inflammatory mediators responsible for the clinical symptoms of the atopic reaction (Gould, HJ. et al., 1991, Clin. Exp. Allergy. 21 (1), 138-147; Riske, F. et al., 1991, J. Biol Chem 266 (17), 11245-11251; Kinet, J.P. et al., 1991, Int. Arch. Allergy Appl. Immunol. 94,51).

Owing to the wide range of diagnostic and therapeutic applications, in recent years interest in synthetic compounds has increased massively, as they are free from biological contaminants, are easily produced, have high chemical stability, are able to bind immunoglobulins non-covalently and interfere with the interaction of these molecules with the receptors or the corresponding antigen.

Patent EP 752 425 describes a peptide, comprising the sequence Arg-Thr-Tyr or Tyr-Thr-Arg, which is able to mimic the affinity of Protein A, extracted from Staphylococcus aureus, for the immunoglobulins, and the use of said peptide in processes of purification of immunoglobulins employing the technique of affinity chromatography.

Patent EP 948 345 describes a peptide comprising one or more sequences Arg-Thr-Tyr or Tyr-Thr-Arg (in which the hydroxyl group of the threonine and the guanidine function of the arginine can be protected by conventional protecting groups) bound to a group that is able to form a dimeric, trimeric or tetrameric peptide. The patent also describes a pharmaceutical composition comprising said peptide for the treatment of pathologies resulting from interaction between immunoglobulins and their receptors, such as, for example, rheumatoid arthritis and allergic reactions.

The activity of the aforementioned peptides for inhibiting the interaction between the immunoglobulins and their receptors has been found to be dose-dependent, and there is an urgent need to identify peptides able to display greater activity at equal dosage.

Surprisingly, a peptide has now been found that possesses, at equal dosage, much greater activity than that of the peptides described in the aforementioned patents.

### Summary of the invention

A first aspect of the present invention relates to a peptide comprising the sequence of formula:

X₁-Arg-Thr-Tyr- (S)

where:
X₁ is the residue of an amino acid, of an amino acid derivative, of a carboxylic acid, or of some other derivative capable of binding covalently to the amino group of arginine, comprising at least one aromatic group.

A second aspect of the present invention relates to a pharmaceutical composition comprising a biologically effective dose of the aforesaid peptide.

A third aspect of the present invention relates to the use of the peptide of the present invention in the manufacture of a pharmaceutical composition for the treatment of allergic diseases.

These and other characteristics of the present invention will be better understood from the description and the examples given below.

### Brief Description of the Figures

Fig. 1 shows a chromatogram obtained by polyacrylamide gel electrophoresis of fractions obtained from purification of rabbit immunoglobulins from raw serum by affinity chromatography on a column prepared by immobilizing the peptide of formula (IB), described later.

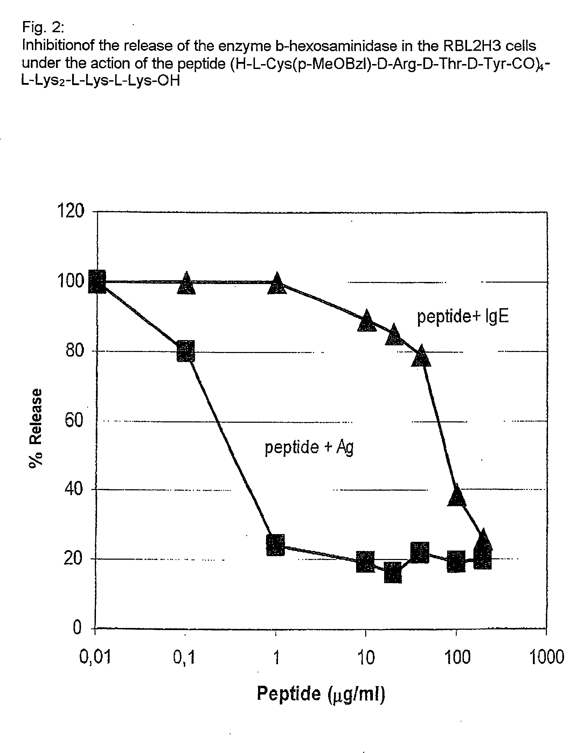

Fig. 2 shows the effects of the aforesaid peptide (lB) on the reaction of release of the enzyme β-hexosaminidase in the RBL2H3 cells. The peptide (IB) was added in increasing doses both to the DNP antigen and to the IgEs. The degranulation assay was performed as described later.

### Definitions

In the present description and in the claims, the following expressions have the following meanings.

The term "amino acid" indicates both of its enantiomeric forms, unless the laevorotatory (L) or dextrorotatory (D) form is expressly indicated.

The expression "amino acid derivative" denotes each of its forms with the free carboxyl group (-COOH), salified (-COO') or amide (-CONH₂) and with the free amino group (-NH₂), salified (-NH₃⁺) or acetylated (CH₃CONH-).

The peptide sequence is understood to be indicated or described, according to the convention familiar to a person skilled in the art, in the direction N → C, unless explicitly stated otherwise.

### Detailed description of the invention

Accordingly, a first object of the present invention is a peptide comprising the sequence of formula:

X₁-Arg-Thr-Tyr- (S)

where:
X₁ is the residue of an amino acid, of an amino acid derivative, of a carboxylic acid, or of some other derivative capable of binding covalently to the amino group of arginine, said residue comprising at least one aromatic group.

According to a first preferred embodiment of the invention, X₁ is selected from the group comprising the following amino acid residues: H-L-Tyr, H-D-Tyr, H-L-Phe, H-D-Phe, H-L-Trp, H-D-Trp, H-L-Phe, H-D-Phe, Ac-L-Tyr, Ac-D-Tyr, Ac-L-Phe, Ac-D-Phe, Ac-L-Trp, Ac-D-Trp, Ac-L-Phe, Ac-D-Phe.

According to a second preferred embodiment of the invention, X₁ is selected from the group comprising the following residues of amino acid derivatives: H-L-Cys(p-MeOBzl), H-L-Cys(p-MeBzl), H-L-Cys(Bzl), H-D-Cys(p-MeOBzl), H-D-Cys(p-Me-Bzl), H-D-Cys(Bzl), Ac-L-Cys(pMeOBzl), Ac-L-Cys(p-Me-Bzl), Ac-L-Cys(Bzl), Ac-D-Cys(p-MeOBzl), Ac-D-Cys(p-Me-Bzl), Ac-D-Cys(Bzl), H-L-Arg(Mts), H-L-Arg(Tos), H-D-Arg(Mts), H-D-Arg(Tos), Ac-L-Arg(Mts), Ac-L-Arg(Tos), Ac-D-Arg(Mts), Ac-D-Arg(Tos), H-L-Trp(CHO), H-D-Trp(CHO), Ac-L-Trp(CHO), Ac-D-Trp(CHO), H-L-Tyr(Bzl), H-L-Tyr(2Cl-Bzl), H-D-Tyr(Bzl), H-D-Tyr(2ClBzl), Ac-L-Tyr(Bzl), Ac-L-Tyr(2Cl-Bzl), Ac-D-Tyr(Bzl), Ac-D-Tyr(2Cl-Bzl), L-Ser(Bzl), D-Ser(Bzl), L-Thr(Bzl), D-Thr(Bzl), L-Asp(Bzl), D-Asp(Bzl), L-Glu(Bzl), D-Glu(Bzl), Ac-L-Ser(Bzl), Ac-D-Ser(Bzl), Ac-L-Thr(Bzl), Ac-D-Thr(Bzl), Ac-L-Asp(Bzl), Ac-D-Asp(Bzl), Ac-L-Glu(Bzl), and Ac-D-Glu(Bzl) where Bzl represents a benzyl group, Mts a 2,4,6-trimethyl phenylsulfonyl group, Tos a tosyl group, 2ClBzl a 2-CI-benzyl group. CHO represents a formyl group.

According to a third preferred embodiment of the invention, X₁ is selected from the group comprising the acyl groups derived from the following carboxylic acids: naphthylacetic, benzoic, 4-methoxybenzoic, 2,4-dichlorobenzoic, 2-nitrobenzoic, 4-chlorobenzoic, phenylacetic, diphenylacetic, (R)-2-phenylpropanoic, (S)-2-phenylpropanoic, (S)-3-phenylpropanoic, (R)-3-phenylpropanoic, (R,S)-2-methyl, 3-phenylbutanoic, (S,R)-2-methyl, 3-phenylbutanoic, (R,R)-2-methyl, 3-phenylbutanoic, (S,S)-2-methyl, 3-phenylbutanoic, (R,S)-2,3-diphenylpentanoic, (R,R)-2,3-diphenylpentanoic, (S,S)-2,3-diphenylpentanoic, (S,R)-2,3-diphenylpentanoic, mono-(9H-fluoren-9-yl-methyl)carbonic acid, monobenzylcarbonic acid, mono-(2-chlorobenzyl)carbonic acid.

According to a preferred aspect, the peptide of the present invention is represented by the following formula:

[X₁-Arg-Thr-Tyr]n-[R]m-[X₂]ₚ (I)

where:
X₁ is the residue of an amino acid, of an amino acid derivative, of a carboxylic acid, or of some other derivative capable of binding covalently to the amino group of arginine, said residue comprising at least one aromatic group,
X₂ is an amino acid, or a polypeptide sequence comprising from 2 to 8 amino acids,
R is an n-valent residue comprising one, two or three amino acids,
m is 0 or 1, n is an integer from 1 to 4, and p is 0 or 1.

In a preferred embodiment, in the aforementioned formula (I) X₁ has the same meanings described previously for sequence (S).

In a preferred embodiment, X₂ is an amino acid selected from the group comprising Lys, Orn, and Dap, or a polypeptide sequence comprising from 2 to 8 amino acids represented by the formula;

(A)q-B

where q is an integer from 1 to 7, A is Gly or Ala, or, when q is at least 2, a polypeptide sequence comprising Gly or Ala, and B is Lys, Orn, or Dap.

In a further preferred embodiment, X₂ is a peptide sequence selected from the group comprising: Gly-Lys, Gly-Orn, Gly-Dap, Ala-Lys, Ala-Orn, Ala-Dap, Gly-Gly-Lys, Gly-Gly-Orn, Gly-Gly-Dap, Gly-Ala-Lys, Gly-Ala-Orn, Gly-Ala-Dap, Ala-Gly-Lys, Ala-Gly-Orn, Ala-Gly-Dap, Ala-Ala-Lys, Ala-Ala-Orn, Ala-Ala-Dap, Gly-Gly-Gly-Lys, Gly-Gly-Gly-Orn, Gly-Gly-Gly-Dap, Ala-Ala-Ala-Lys, Ala-Ala-Ala-Orn, Ala-Ala-Ala-Dap, Gly-Gly-Gly-Gly-Lys, Gly-Gly-Gly-Gly-Orn, Gly-Gly-Gly-Gly-Dap, Gly-Gly-Gly-Gly-Gly-Lys, Gly-Gly-Gly-Gly-Gly-Orn, Gly-Gly-Gly-Gly-Gly-Dap, Gly-Ala-Gly-Ala-Gly-Lys, Gly-Ala-Gly-Ala-Gly-Orn, Gly-Ala-Gly-Ala-Gly-Dap, Ala-Gly-Ala-Gly-Ala-Gly-Ala-Lys, Ala-Gly-Ala-Gly-Ala-Gly-Ala-Orn, Ala-Gly-Ala-Gly-Ala-Gly-Ala-Dap.

In the aforementioned formula (I), when n is equal to 2 and R represents a divalent group (dimer), group R is preferably selected from the group comprising lysine (Lys), ornithine (Orn), diaminopropionic acid (Dap) and diaminobutyric acid (Dab).

The peptide can then be represented by the following formulae:

[X₁-Arg-Thr-Tyr]₂-Lys-[X₂]ₚ (I)-2a

[X₁-Arg-Thr-Tyr]₂-Orn-[X₂]ₚ (I)-2b

[X₁-Arg-Thr-Tyr]₂-Dap-[X₂]ₚ (I)-2c

[X₁-Arg-Thr-Tyr]₂-Dab-[X₂]ₚ (I)-2d

where X₁ and X₂ and p have the meanings stated previously.

In the aforementioned formula (I), when n is equal to three and R represents a trivalent group (trimer), group R is preferably selected from the group comprising a dipeptide of the type (Lys-Lys), (Orn-Orn) or (Dap-Dap) or (Dab-Dab).

The peptide can then be represented by the following formulae:

[X₁-Arg-Thr-Tyr]₃-(Lys-Lys)-[X₂]ₚ (I)-3a

[X₁-Arg-Thr-Tyr]₃-(Orn-Orn)-[X₂]ₚ (I)-3b

[X₁-Arg-Thr-Tyr]₃-(Dap-Dap)-[X₂]ₚ (I)-3c

[X₁-Arg-Thr-Tyr]₃-(Dab-Dab)-[X₂]ₚ (I)-3d

where X₁, X₂ and p have the meanings stated previously.

In the aforementioned formula (I), when n is equal to four and R represents a tetravalent group (tetramer), group R is preferably selected from the group comprising a branched tripeptide of formula Lys(εLys)-Lys, Orn(δOrn)-Orn, Dap(βDap)-Dap, and Dab(γDab)-Dab, indicating that the residue in parentheses is bound to the amino function in position ε, δ, β or γ, respectively, of the residue preceding it.

This notation can also be shown as:

Lys(εLys)-Lys = Lys₂-Lys

Orn(δOrn)-Orn - Orn₂-Orn

Dap(βDap)-Dap = Dap₂-Dap

Dab(γDab)-Dab = Dab₂-Dab

The peptide can then be represented by the following formulae:

[X₁-Arg-Thr-Tyr]₄-(Lys₂-Lys)-[X₂]ₚ (I)-4a

[X₁-Arg-Thr-Tyr]₄-(Orn₂-Orn)-[X₂]p (I)-4b

[X₁-Arg-Thr-Tyr]₄-(Dap₂-Dap)-[X₂]ₚ (I)-4c

[X₁-Arg-Thr-Tyr]₄-(Dab₂-Dab)-[X₂]ₚ (I)-4d

where X₁, X₂, and p have the meanings stated previously.

In a particularly preferred embodiment, group R comprises the tripeptide Lys(εLys)-Lys, group X₁ is selected from those stated, preferably from carboxylic acids and acetylated amino acids, group X₂ is selected from the group L-Lys-OH and the tripeptide Arg-Thr-Tyr in which all the amino acids have configuration D.

The resultant molecule possesses a tetrameric structure with 4 free or acetylated terminal NH₂ groups and a lysine residue at the C terminus which, when the terminal groups are acetylated, can be useful for immobilizing the molecule itself on pre-activated solid phases.

Specific examples of peptides of the present invention are listed hereunder.

IA (H-D-Cys(p-MeOBzl)-D-Arg-D-Thr-D-Tyr)₄-L-Lys₂-L-Lys-L-Lys-OH

IB (H-L-Cys(p-MeOBzl)-D-Arg-D-Thr-D-Tyr)₄-L-Lys₂-L-Lys-L-Lys-OH

IC (H-D-Cys(p-MeOBzl)-L-Arg-L-Thr-L-Tyr)₄-L-Lys₂-L-Lys-L-Lys-OH

ID (H-L-Cys(p-MeOBzl)-L-Arg-L-Thr-L-Tyr)₂-Lys-Gly-Lys-OH

IE H-L-Cys(p-MeOBzl)-D-Arg-D-Thr-D-Tyr-Gly-L-Ala-Gly-L-Ala-Gly-L-

Orn-OH

IF (Ac-L-Cys(p-MeOBzl)-L-Arg-L-Thr-L-Tyr)₄-L-Lys₂-L-Lys-L-Lys-OH

IG H-L-Cys(p-MeOBzl)-D-Arg-D-Thr-D-Tyr-Gly-L-Ala-Gly-L-Ala-Gly-L-

Lys-OH

IH (H-L-Cys(p-MeOBzl)-L-Arg-L-Thr-L-Tyr)₂-Lys-Lys*(CO-L-Tyr-L-Thr-

L-Arg-L-Cys(p-MeOBzl))-OH

IL (H-L-Cys(p-MeOBzl)-L-Arg-L-Thr-L-Tyr)₄-L-Orn₂-L-Orn-L-Orn-OH

IM (H-L-Cys(p-MeOBzl)-L-Arg-L-Thr-L-Tyr)₂-Orn-Gly-OH

IN (Ac-L-Cys(p-MeOBzl)-L-Arg-L-Thr-L-Tyr)₄-L-Orn₂-L-Orn-L-Orn-OH

lO (H-L-Cys(p-MeOBzl)-L-Arg-L-Thr-L-Tyr)₂-L-Orn-L-Orn*(CO-L-Tyr-L-

Thr-L-Arg-L-Cys(p-MeOBzl))-OH

IP (Ac-L-Cys(p-MeOBzl)-D-Arg-D-Thr-D-Tyr)₄-L-Lys₂-L-Lys-L-Lys-OH

IQ (Ac-D-Cys(p-MeOBzl)-L-Arg-L-Thr-L-Tyr)₄-L-Lys₂-L-Lys-L-Lys-OH

IR (Ac-L-Cys(p-MeOBzl)-L-Arg-L-Thr-L-Tyr)₄-L-Lys₂-L-Lys-L-Lys-NH₂

IS (Ac-D-Cys(p-MeOBzl)-D-Arg-L-Thr-D-Tyr)₄-L-Lys₂-L-Lys-L-Lys-NH₂

IT (Ac-D-Cys(p-MeOBzl)-L-Arg-D-Thr-L-Tyr)₄-L-Lys₂-L-Lys-L-Lys-OH

IU (Ac-L-Cys(p-MeOBzl)-D-Arg-D-Thr-L-Tyr)₄-L-Lys₂-L-Lys-L-Lys-OH

The peptides of formula (I) can be prepared according to the usual techniques for preparation of peptides, both in the liquid phase and in the solid phase, comprising the protection, coupling and deprotection of amino acids.

Preparation by the technique of solid phase synthesis is preferably carried out manually using simple apparatus, such as plastic tubes equipped with filtering diaphragms and stirrers, or by means of automatic synthesizers, an example of which is the model 433A made by Applied Biosystems (Foster City, Ca, USA).

Advantageously, preparation is carried out in accordance with the usual methods known by a person skilled in the art, e.g. the methods described by Atherton, E. & Sheppard, R.C. (1989) Solid Phase Peptide Synthesis: A Practical Approach. IRL Press, Oxford, England and by Bodansky, M. & Bodansky, A. (1994) The Practice of Peptide Synthesis, 2nd ed. Springer, Berlin. As an example, it is possible to use the methods of preparation described in patent EP 752 425.

Preferably, the functional group of the first amino acid used for anchoring to the resin is such that it can be hydrolysed by treatment with strong acids such as trifluoroacetic acid (TFA), whereas the second functional group is provided with suitable protection that can be removed after anchoring to the resin. If the first amino acid is trifunctional, e.g. Lys or Orn or Dap or Dab, the functions protected during anchoring to the resin will be two in number.

Protection can be carried out by methods that are known in the prior art, for example using the Fmoc group (Atherton, E, & Sheppard, and Bodansky, M. & Bodansky, A).

During the stage of coupling and sequential deprotection of Tyr, Thr, Arg, the tyrosine is preferably protected at the phenolic hydroxyl group with a tert-butyl group (tBu, Field G.B. and Noble R.L. 1990 Int J Pept Protein Res; 35: 161-214; "The practice of paptide synthsis, 2nd ed., by M. Bodansky and A., Bodansky, Springer-Verlag, New York, 1995), the threonine is preferably protected at the hydroxyl group with a similar tert-butyl group (tBu, Field, 1997; Bodansky, 1995), and the arginine is preferably protected with protection by a labile acid, for example pentamethylchroman (Pmc, Field, 1997; Bodansky, 1995) or pentamethyldihydrobenzofuran (Pbf, Field, 1997; Bodansky, 1995).

The terminal amino groups of the resultant peptide can be acetylated subsequent to preparation of the peptide, or the appropriate amino acid residue, acetylated beforehand, can be introduced directly.

Detachment of the various labile-acid protecting groups of the peptide preferably takes place simultaneously with detachment of the peptide itself from the supporting resin.

The peptides of the present invention are able to bind non-covalently to at least one immunoglobulin. The peptides of the present invention can be used in the treatment of diseases involving immunoglobulins.

A further aspect of the present invention relates to a pharmaceutical composition comprising a biologically effective dose of the peptides described above and the use of said peptides for the production of a pharmaceutical composition for therapeutic applications and in particular for the treatment of allergic diseases.

Preferably, the pharmaceutical compositions of the present invention are prepared as suitable dosage forms comprising a biologically effective dose of at least one of the aforementioned peptides and at least one pharmaceutically acceptable excipient.

Examples of suitable dosage forms are tablets, capsules, coated tablets, granules, solutions and syrups for oral administration; creams, ointments and medicated patches for topical administration; suppositories for rectal administration and sterile solutions for administration by injection, as aerosols or ophthalmic administration.

The dosage forms can also contain other conventional ingredients such as preservatives, stabilizers, surfactants, buffers, salts for controlling osmotic pressure, emulsifiers, sweeteners, colourants, flavouring agents and the like.

If required for particular treatments, the pharmaceutical composition of the present invention can contain other pharmacologically active ingredients whose concomitant administration is therapeutically useful.

The amount of the aforementioned peptides in the pharmaceutical composition of the present invention can vary over a wide range in relation to known factors, for example the severity of the disease, the patient's body weight, the dosage form, the chosen route of administration, the number of administrations per day and the efficacy of the peptide selected. However, the optimum amount can easily be determined by a person skilled in the art.

The dosage forms of the pharmaceutical composition of the present invention can be prepared by techniques that are familiar to a person skilled in the art and comprise mixing, granulation, compression, dissolution, sterilization and the like.

The solid phase synthesis of the peptides of the present invention has been carried out both by a manual method, and using the automatic synthesizer model 433 A, software version 1.2, made by Applied Biosystems (Foster City, Ca, USA), in accordance with the methods of synthesis recommended by the supplier, based on methods described in the literature (Field *loc. cit.,* Bodansky, loc. *cit.).*

In the examples given below the abbreviation "Cat. No." stands for "catalogue number".

The examples given below are provided for better description of the invention but do not limit it in any way.

### EXAMPLE 1

### Solid phase preparation of a peptide of formula (I) in which X₁ is an L-cysteine residue protected on the thiol group with para-methoxybenzyl (pMeOBzl), the amino acids Arg, Thr and Tyr are in configuration D, group R is the branched tripeptide L-Lys₂-L-Lys, X₂ is the amino acid L-Lys-OH.

The molecule can be represented schematically with the following formula:

IB (H-L-Cys(p-MeOBzl)-D-Arg-D-Thr-D-Tyr-)₄-L-Lys₂-L-Lys-L-Lys-OH

Preparation of the peptide was carried out manually by solid phase synthesis in accordance with the Fmoc/HOBt/HBTU methodology described hereunder.

1.78 g of resin for solid phase synthesis of the polystyrene type (polystyrene, 1% divinylbenzene, 100-200 mesh), derivatized with the hydroxymethylphenoxyl group, substitution 0.84 meq/g (Novabiochem, Laufelfingen, Switzerland, Cat. No. 01-64-0014) was packed in a glass reactor of 300 ml equipped with a type R3 sintered glass filter. The resin was washed 3 times for 5 minutes with 50 ml of a 3:2 (v/v) mixture of DCM/DMF (DCM, DiChloroMethane, LabScan, Dublin, Ireland, Cat. No. H6508L; DMF, DiMethylFormamide, LabScan, Dublin, Ireland, Cat. No. H33H11X).

7.02 g (15 mmol) of Fmoc-Lys(Boc)-OH (Novabiochem, Laufelfingen, Switzerland, Cat. No. 04-12-1026) was dissolved in 30 ml of a 3:2 (v/v) mixture of DCM/DMF, then 7.5 ml of a 1M solution of DCC in NMP (DCC/NMP, DiCyclohexyl-Carbodiimide/N-Methyl-Pyrrolidone, Sigma-Aldrich, Milan, Italy, Cat. No. 36651) was added. The resultant solution was stirred for 20 minutes at room temperature and then filtered to remove the white precipitate of dicyclohexylurea (DCU). After adding 5.4 ml of a 0.1 M solution of dimethylaminopyridine (DMAP, Sigma-Aldrich, Milan, Italy, Cat. No. 39408) in DMF, the solution was poured into the reactor containing the resin, and stirred for one hour at room temperature. The solution of amino acid was drained and the resin was washed 3 times with 50 ml of DMF. The resin was then treated with 50 ml of 0.5M solution of acetic anhydride in DMF (Ac₂O, Sigma-Aldrich, Milan, Italy, Cat. No. 45830) containing 2.5 mmol of DMAP for 1 hour at room temperature. After draining the solution, the resin was washed 3 times with 50 ml of DMF.

The Fmoc group was removed from the amino group of the lysine by treatment with 50 ml of solution of piperidine/DBU/DMF 2:2:96 v/v/v (DBU, 1,8-diazabicyclo[5.4.0]undec-7-ene, Sigma-Aldrich, Milan, Italy, Cat. No. 33482: piperidine: Sigma-Aldrich, Milan, Italy, Cat. No. 80641) for 30 minutes at room temperature. The deprotection solution containing the Fmoc-piperidine adduct was filtered and the resin was washed 3 times with 50 ml of DMF. The deprotection solution and the three washings with DMF were combined and were used for obtaining an estimate of the reaction yield. A small aliquot of 100 µL of the solution (total volume 200 ml) was diluted 100-fold with DMF and after zeroing with DMF, its absorbance at 301 nm was determined (1 cm cuvette) using a spectrophotometer. The concentration of the adduct was calculated using a value ε_{301 nm} = 7.8 mM⁻¹ cm⁻¹. From the absorbance value of 0.563 it was calculated that (0.563/7.8 mM⁻¹ cm⁻¹) x 0.200L x 100 x 1 cm = 1.44 mmol of Fmoc-L-Lys(Boc)-OH was bound to the resin.

4.43 g of Fmoc-L-Lys(Fmoc)-OH (7.5 mmol, Novabiochem, Laufelfingen, Switzerland, Cat. No. 04-12-1085) was dissolved in 15 ml of DMF. 16.7 ml of a 0.45 M solution of HBTU/HOBt in DMF, equivalent to 7.5 mmol of HBTU and 7.5 mmol of HOBt (HBTU, O-(benzotriazol-1-yl)-N,N,N',N'-tetramethyluronium hexafluorophosphate, Sigma-Aldrich, Milan, Italy, Cat. No. B2778; HOBt: N-hydroxybenzotriazole, Sigma-Aldrich, Milan, Italy, Cat. No. 157260) was added to the solution obtained. After adding 2.62 ml (15 mmol) of diisopropyl-ethylamine (DIEA, Sigma-Aldrich, Milan, Italy, Cat. No. 03440), the solution was stirred for 3 minutes and then poured into the reactor containing the resin, and the suspension was stirred for one hour at room temperature. The resin was drained under vacuum and washed 3 times with 50 ml of DMF.

Removal of the Fmoc group was carried out with 50 ml of piperidine/DBU/DMF 2:2:96 v/v/v for 30 minutes at room temperature, washing a further 3 times with 50 ml of DMF. The amount of Fmoc-piperidine released in the deprotection solution was determined as described previously, obtaining a value of 2.82 mmol (theoretical 2.88 mmol) for a yield of 98.0%.

To obtain the second level of branching, a second reaction was carried out with Fmoc-L-Lys(Fmoc)-OH. 8.86 g (15 mmol) of amino acid was dissolved in 30 ml of DMF. Activation was effected by adding 33.4 ml of 0.45M HBTU/HOBt solution in DMF (15 mmol of HBTU and 15 mmol of HOBt) and 5.24 ml of DIEA (30 mmol). After 3 minutes, the solution was poured into the reactor containing the resin, and stirred for one hour at room temperature. The resin was drained under vacuum and washed 3 times with 50 ml of DMF.

Removal of the Fmoc group was carried out again by treating the resin with 100 ml of piperidine/DBU/DMF 2:2:96 v/v/v for 30 minutes at room temperature. The reaction yield was determined as described previously, obtaining a value of 97.5% (5.50/5.64 mmol of Fmoc-piperidine).

For reaction of D-tyrosine with the four free amino groups of the tetrapeptide L-Lys₂-L-Lys-L-Lys(Boc)-OH bound to the resin, 13.77 g (30 mmol) of Fmoc-D-Tyr(tBu)-OH (Novabiochem, Laufelfingen, Switzerland, Cat. No. 04-12-1037) was dissolved in 60 ml of DMF and activated as described previously for three minutes with 66.8 ml of 0.45M solution of HBTU/HOBt in DMF and 10.48 ml (60 mmol) of DIEA. The solution was poured into the reactor containing the resin, stirring for one hour at room temperature, and then the solution of amino acid was drained under vacuum. The resin was washed 3 times with 100 ml of DMF. To remove the Fmoc group, the resin was again treated with 100 ml of piperidine/DBU/DMF 2:2:96 v/v/v for 30 minutes at room temperature. The estimated reaction yield was 97.0% (5.33/5.50 mmol).

For reaction of D-threonine with the amino group of D-tyrosine, 11.91 g (30 mmol) of Fmoc-D-Thr(tBu)-OH (Novabiochem, Laufelfingen, Switzerland, Cat. No. 04-12-1000) was dissolved in 60 ml of DMF and activated with 66.8 ml of 0.45M solution of HBTU/HOBt in DMF and 10.48 ml (60 mmol) of DIEA. After three minutes, the solution was poured into the reactor containing the resin, stirring for one hour at room temperature, then the resin was drained and washed 3 times with 100 ml of DMF. Removal of the Fmoc group was carried out by treating the resin with 100 ml of piperidine/DBU/DMF 2:2:96 v/v/v for 30 minutes at room temperature. The reaction yield determined was 97.4% (5.19/5.33 mmol).

For reaction of D-arginine, 19.44 g (30 mmol) of Fmoc-D-Arg(Pbf)-OH (Novabiochem, Laufelfingen, Switzerland, Cat. No. 04-12-1145) was dissolved in 60 ml of DMF and activated for three minutes with 66.8 ml of 0.45M solution of HBTU/HOBt in DMF and 10.48 ml (60 mmol) of DIEA. The solution was poured into the reactor containing the resin, stirring for one hour at room temperature, then the resin was drained and washed 3 times with 100 ml of DMF. Removal of the Fmoc group was effected by treating the resin with 100 ml of piperidine/DBU/DMF 2:2:96 v/v/v for 30 minutes at room temperature. The reaction yield determined was 98.1 % (5.09 mmol/5.33 mmol).

For reaction of the protected L-cysteine, 10.23 g (30 mmol) of Boc-L-Cys(p-MeOBzl)-OH (Chem-Impex, Wood Dale, IL, USA, Cat. No. 01342) was dissolved in 60 ml of DMF and activated with 66.8 ml of 0.45M solution of HBTU/HOBt in DMF and 10.48 ml (60 mmol) of DIEA. The solution was poured into the reactor containing the resin, stirring for 90 minutes at room temperature, and then the resin was washed with 100 ml of DMF, 100 ml of DCM, 100 ml of CH₃OH (MeOH, LabScan, Dublin, Ireland, Cat. No. C2517) and 100 ml of ethyl ether (Et₂O, LabScan, Dublin, Ireland, Cat. No. A3509E). The resin was dried under vacuum and weighed. 8.52 g of dry resin was obtained.

The reaction of detachment of the peptide from the resin and removal of the protecting groups was carried out by treating the resin for 3 hours with the following mixture: 100 ml trifluoroacetic acid (Sigma-Aldrich, Milan, Italy, Cat. No. 91700); 5.0 ml of H₂O, 5.0 ml of thioanisole (Sigma-Aldrich, Milan, Italy, Cat. No. 88470), 7.5 g of phenol (Sigma-Aldrich, Milan, Italy, Cat. No. 77612) and 2.5 ml of triisopropylsilane (TIS, Sigma-Aldrich, Milan, Italy, Cat. No. 92095) (83:4:5:6:2, w/w/w/w/w).

At the end of reaction, the resin was removed by filtration and the resultant solution was concentrated to approx. 40 ml using a rotary evaporator.

The peptide of formula (IB) was precipitated by pouring the acid solution dropwise into 200 ml of cold ethyl ether. The precipitate was separated by centrifugation at 3500 rpm for 15 minutes and the solvent was discarded. The precipitate was washed again with 200 ml of cold ethyl ether and was then dissolved in 50 ml of H₂O/CH₃CN/TFA (50:50:0.1) and lyophilized. After lyophilization, 4.26 g of raw product was recovered in the form of trifluoroacetate salt. Taking into account that the molecule can hold 8 molecules of TFA, the yield based on raw product, calculated from the initial 1.44 mmol, was 73.4%.

The peptide of formula (IB) was characterized by analytical RP-HPLC and MALDI-TOF mass spectrometry. For the HPLC analysis, 10 µg of product was injected in a column C18 Jupiter 250x4.6 mm ID, 5 µm (Phenomenex, Torrance, CA, USA) adjusted to a flow of 1 ml/min with 5% CH₃CN in H₂O, 0.1 % TFA (CH₃CN, acetonitrile, LabScan, Dublin, Ireland, Cat. No. C2503). Elution was carried out with a gradient from 5% to 65% of CH₃CN, 0.1 % TFA for 35 minutes. The HPLC system used comprised a Merck low-pressure gradient pump, a Shimadzu photodiode detector for acquisition at all wavelengths from 190 nm to 800 nm and a Rheodyne valve with a 20 µL loop. According to the HPLC analysis (225 nm) the product had an estimated purity of 75% and Rt: 29.7 min.

Approximately 1 µg of peptide of formula (IB) was analysed by mass spectrometry to determine the molecular weight. This determination was carried out using a Kratos MALDI-TOF spectrometer of the MALDI III type operating in "reflectron" mode. The matrix used for the analysis was caffeic acid (Sigma-Aldrich, Milan, Italy, Cat. No. C0625; 20 mg/ml in H₂O/CH₃CN/TFA, 50:50:0.1, 1 µL) mixed with the sample solution and left to dry. 200 spectra were acquired with the laser power at 60% of maximum, and they were averaged. Calibration was performed using a solution of Porcine Insulin (MW: 5734.5 amu; Sigma-Aldrich, Milan, Italy, Cat. No. I6279) at a concentration of 0.57 mg/ml in H₂O/CH₃CN/TFA, 50:50:0.1. The experimental value of molecular weight obtained was in excellent agreement with the calculated value (MW_{exp./calc.}: 3106.8/3106.2 amu).

A 0.6 g aliquot of the raw peptide was purified by preparative RP-HPLC using a column RP-18 25x2.2 cm ID, 10 µm (Phenomenex, Torrance, CA, USA). The column was installed in a high-pressure HPLC system of the LC8 type equipped with a double reciprocating pump, high-pressure gradient device, detector for wavelength varying in the range 190-800 nm, a 10 ml loop and a controller (Shimadzu, Milan, Italy) and adjusted to a flow of 20 ml/min with 100% of buffer A. Buffer A comprises 15% CH₃CN/H₂O 0.1 % TFA; buffer B comprises 80% CH₃CN/H₂O, 0.1% TFA. The 0.6 g of product was dissolved in 10 ml of 20% CH₃CN/H₂O, 0.1 % TFA and purified in 10 passes, injecting 1.0 ml of solution in each pass. The gradient applied was as follows:

| | | | | | |
|---|---|---|---|---|---|
| Time | 0 | 10 | 65 | 75 | 80 |
| %B | 0 | 0 | 100 | 100 | 0 |

The eluate was monitored at wavelength of 280 nm and fractionated into 30 tubes between 30 and 60 minutes. The fractions containing the purified product were combined, lyophilized and analysed by RP-HPLC in the conditions stated above. The purified product had an estimated purity of 99% and Rt of 29.6 min. After lyophilization, 148.8 mg of pure product was recovered, for a purification yield of 24.8%.

The following peptides were prepared similarly:

IC (H-D-Cys(p-MeOBzI)-L-Arg-L-Thr-L-Tyr)₄-L-Lys₂-L-Lys-L-Lys-OH

ID (H-L-Cys(p-MeOBzl)-L-Arg-L-Thr-L-Tyr)₂-Lys-Gly-Lys-OH

IE H-L-Cys(p-MeOBzI)-D-Arg-D-Thr-D-Tyr-Gly-L-Ala-Gly-L-Ala-Gly-L-Orn-OH

IF (Ac-L-Cys(p-MeOBzI)-L-Arg-L-Thr-L-Tyr)₄-L-Lys₂-L-Lys-L-Lys-OH

IG H-L-Cys(p-MeOBzI)-D-Arg-D-Thr-D-Tyr-Gly-L-Ala-Gly-L-Ala-Gly-L-Lys-OH

IH (H-L-Cys(p-MeOBzI)-L-Arg-L-Thr-L-Tyr)₂-Lys-Lys*(CO-L-Tyr-L-Thr-L-Arg-L-Cys(p-MeOBzl))-OH

IL (H-L-Cys(p-MeOBzI)-L-Arg-L-Thr-L-Tyr)₄-L-Orn₂-L-Orn-L-Orn-OH

IM (H-L-Cys(p-MeOBzI)-L-Arg-L-Thr-L-Tyr)₂-Orn-Gly-OH

IN (Ac-L-Cys(p-MeOBzI)-L-Arg-L-Thr-L-Tyr)₄-L-Orn₂-L-Orn-L-Orn-OH

lO (H-L-Cys(p-MeOBzI)-L-Arg-L-Thr-L-Tyr)₂-L-Orn-L-Orn*(CO-L-Tyr-L-Thr-L-Arg-L-Cys(p-MeOBzl))-OH

### EXAMPLE 2

### Immobilization of the peptide of formula (IB) on preactivated resin CH-Sepharose-4B and purification of immunoglobulins from rabbit serum by affinity chromatography.

The peptide of formula (IB) prepared in Example 1 (10 mg) was dissolved in 5 ml of aqueous solution of sodium bicarbonate 0.1 M, NaCl 0.5 M, pH 8.5 and then added to 0.5 g of activated resin CH-Sepharose-4B (Amersham Biosciences, Uppsala, Sweden, Cat. No. 17-0490-01), a preactivated support for affinity chromatography for the direct coupling of peptides and proteins. The suspension was stirred for 24 hours and the extent of coupling was monitored by taking samples of the reaction mixture, at various times, followed by analysis by RP-HPLC.

Approximately 90-95% of the initial peptide is found to be bound covalently to the resin after 24 hours. The derivatized resin is incubated with 0.1 M Tris, pH 8.5 to block any sites of the resin that are still reactive, and is then packed in a glass column 100 x 0.6 cm I.D. with a variable piston (OMNIFIT, Cat. No. 006CC-06-10-FF).

For the purification of immunoglobulins from serum, the column was equilibrated with a buffer 50 mM Bis-Tris (Sigma, Cat. No. B9754), pH 6.5, to a flow of 1 ml/min, monitoring the eluent at 280 nm. Raw rabbit serum (Sigma, Cat, No. R9133) was loaded in the column and, after elution of the material that was not bound, the eluent was changed to acetic acid 0.1 M, pH 3.0, for elution of the adsorbed material.

The material eluted from the column was collected, neutralized with Tris 1 M, and characterized by analysis by polyacrylamide gel electrophoresis.

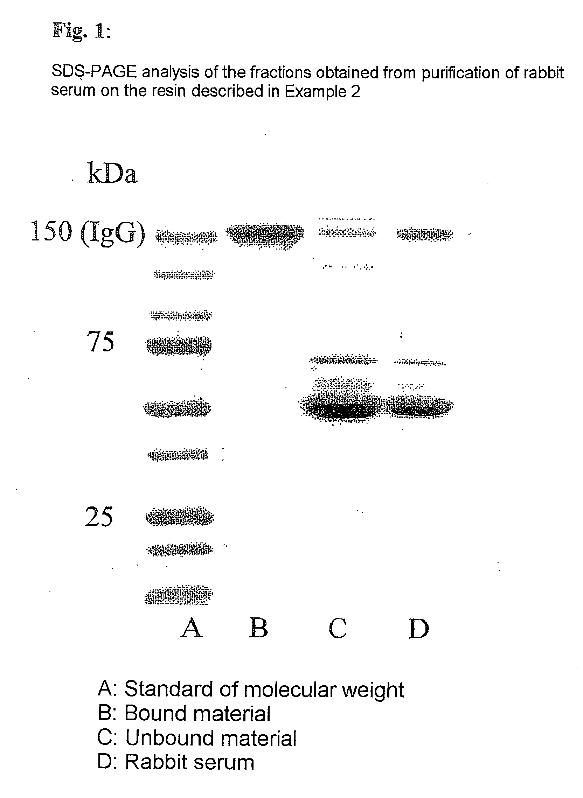

As clearly shown by the electrophoretic analysis in Fig. 1, the column was able to retain the immunoglobulin fraction of the raw serum, as demonstrated by Western Blot analysis, whereas the albumin was eluted to the dead volume of the column.

Similar results were obtained with raw human serum (Sigma-Aldrich, Cat. No. H1388) and raw murine serum (Sigma-Aldrich, Cat. No. M5905). In this case too, the column was able to retain the immunoglobulins (IgG, IgM, IgA and IgE), as shown by Western Blot analysis, whereas the albumin was recovered in the material that was not retained.

Similar results, in terms of immobilization and purification, were obtained with the following peptides:

ID (H-L-Cys(p-MeOBzI)-L-Arg-L-Thr-L-Tyr-CO)₂-Lys-Gly-Lys-OH

IE H-L-Cys(p-MeOBzI)-D-Arg-D-Thr-D-Tyr-Gly-L-Ala-Gly-L-Ala-Gly-L-Orn-OH

### EXAMPLE 3

### Determination "in vitro" of the anti-allergic activity of the peptide of formula (lB) and of the peptide of formula (H-D-Arg-D-Thr-D-Tyr-)₄-L-Lys₂-L-Lys-Gly-OH (comparative peptide) in the assay for degranulation of the RBL 2H3 cells.

The RBL 2H3 cell line, rat basophils that express the receptor with high affinity for the immunoglobulins of class E (FcεRl), is widely used for investigating allergic reactions, because in this cell line the chemical mediators of inflammation are released as a result of allergen specific-IgE-FcεRI binding.

Release of the enzyme β-hexose-aminidase, which is present together with histamine in the granules of mast cells and basophils, can be used as an index of cellular degranulation (Tanaka et al., 1991, Chem. Pharm. Bull, 39, 2072-2076; McCloskey et al., 1988, Proc. Natl. Acad. Sci., USA, 85, 7260-7264).

The activity of the enzyme β-hexose-aminidase, released by the stimulated RBL 2H3 cells, was determined by the method described by Yamanishi et al., 1995, Jour. Biotech. Biochem., 59 (7), 1272-1275, which envisages measurement of the enzymatic activity by measuring the absorbance of the product p-nitrophenol generated by the synthetic substrate p-nitrophenyl-N-acetyl-β-D-glucosamide. 1x10⁵ RBL 2H3 cells, trypsinized from plates at confluence, were resuspended in a volume of 80 µl of the culture medium (MEM + 10% inactivated fetal bovine serum) and distributed in each well of the 96-well plates (Costar). After 24 hours, 20 µl of medium containing anti-2,4-dinitrophenol monoclonal IgEs (DNP, Sigma, Cat. No. D-8406) was added to each well.

The microplates were incubated at 37°C for one hour and then washed three times with 100 µl of HBS buffer solution (10 mM HEPES, 135 mM NaCl, 5 mM KCI, 1 mM CaCl₂, 0.5 mM MgCl₂, 5.6 mM glucose, 0.1 % BSA, pH 7.4). After the washings, 100 µl of HBS containing the 2,4-dinitrophenol antigen conjugated to BSA (Sigma, Cat. No. A-6661) was added. As a control, 100 µl of HBS containing 1% Triton X-100 was added to some wells to determine the total amount of enzyme contained in the cellular granules.

After further incubation at 37°C for two hours, 30 µl of cellular supernatant or of cellular lysate was transferred to a new microplate, to which 50 µl of sodium citrate buffer at pH 4.5, containing 4 mM p-nitrophenyl-N-acetyl-β-D-glucosaminide (Sigma, Cat. No. N9376), a specific substrate for the enzyme β-hexose-aminidase, had been added beforehand. After incubation for two hours at 37°C, the reaction was stopped by adding 100 µl of glycine solution 0.2 M, pH 10.7. The enzymatic activity was determined by measuring the absorbance at 405 nm with a Microplate Reader Model 3550 EIA (Bio-Rad).

In preliminary experiments the immunoglobulins E and the antigen were added at various concentrations in order to determine the optimum concentrations for induction of the reaction of enzymatic release. The conditions identified experimentally correspond to 0.5 µg/ml of IgE and 0.05 µg/ml of antigen DNP conjugated to BSA.

To verify the capacity of the peptides of formula IB and of comparative peptides for inhibiting the degranulation of the RBL 2H3 cells, the peptides were added in the assay together with the antigen at concentrations between 0.1 and 500 µg/ml.

The comparative peptide was found to be able to reduce 50% of the cellular degranulation at a concentration of 10 µg/ml (4.6 pM). The peptide of formula IB was found to be able to reduce 50% of the cellular degranulation at a concentration as low as 1 µg/ml (approx. 0.3 µM), showing activity approx. 15 times greater than the activity of the comparative peptide. The full results, expressed as percentage inhibition of the degranulation reaction, are shown in the following Table 1.

**TABLE 1**

| Concentration of Peptide (µg/ml) | Peptide IB | Comparative peptide |
|---|---|---|
| 0.1 | 20 | 0 |
| 1 | 76 | 40 |
| 10 | 80 | 49 |
| 50 | 82 | 59 |
| 100 | 85 | 62 |
| 500 | 90 | 70 |

Results similar to the results obtained with peptide IB were obtained with the following peptides:

ID (H-L-Cys(p-MeOBzl)-L-Arg-L-Thr-L-Tyr)₂-Lys-Gly-Lys-OH

IF (Ac-L-Cys(p-MeOBzl)-L-Arg-L-Thr-L-Tyr)₄-L-Lys₂-L-Lys-L-Lys-OH

### EXAMPLE 4

### Determination "in vivo" of the anti-allergic activity of the peptide of formula (IB) and of the peptide of formula (H-D-Arg-P-Thr-D-Tyr)₄-L-Lys₂-L-Lys-Gly-OH (comparative peptide) using passive cutaneous anaphylaxis in the rat as the experimental animal model.

Passive cutaneous anaphylaxis in the rat was induced according to the procedure described by Ovary et al., 1952, Int. Arch. Allergy Appl. Immunol. 3:293-301.

Rat ascitic fluid containing specific IgE against the ovalbumin antigen was injected in the shaved back of male rats of the CD1 species (Charles River, Italy). Ascitic fluid at different dilutions (from 20 to 20 000 times in PBS) was applied by intradermal injections at a volume of 50 µl, permitting the IgEs present in the ascitic fluid to bind to the FcεRs of the local mast cells, sensitizing them.

On the next day, 1 mg of ovalbumin and the dye Evans Blue at a concentration of 2.0%, dissolved in 1 ml of PBS, were injected intravenously.

After 30 minutes, the appearance, on the rats' backs, of stained areas in the dermis (wheals) with extent and intensity of staining proportional to the quantity of antigen-specific IgEs injected with the ascitic fluid, was observed. The experiment included a negative control, represented by the intradermal injection of 50 µl of PBS without ascitic fluid, and there was no corresponding appearance of staining. The rats were sacrificed and comparative evaluation of the dimensions and intensity of coloration of the wheals that developed was carried out by densitometric analysis of colour photographs of the animals taken with a single camera, a single film and in the same conditions of exposure times, lighting and orientation. The densitometric analyses were performed with the aid of the IMAGE PRO-PLUS software (Media Cybernetics, Inc.).

On the basis of the results obtained in the IgE-FcεR linear binding assay (absence of inhibitors), selecting as reference point the value of dilution of the ascitic fluid at which the most intense inflammatory reaction was observed (the wheal with most intense staining, and consequently the maximum signal on the densitometer), an assay of inhibition of the allergic reaction was performed using peptide lB and the comparative peptide.

For this purpose, 25 µl of ascitic fluid containing IgE suitably diluted was incubated with 25 µl of PBS containing different doses of the test compounds (0.025, 0.25, 2.5, 25, 250 and 2500 µg). A single rat was used for each compound. The back of each rat was shaved and was then subdivided into 8 sections by making marks to delimit the zones in which the injections were made. The mixture (50 µl) containing the ascitic fluid and the compounds at the various doses was administered intradermally at each selected point. An injection of 25 µl of ascitic fluid suitably diluted and incubated with 25 µl of PBS was used as the positive control, and 50 µl of PBS was used as the negative control.

After 24 hours, the antigen was injected together with the Evans Blue dye as previously described and thirty minutes later, corresponding to the injection sites on the rats' backs, the appearance of coloured wheals was observed, with intensity and dimensions markedly less than in the zones in which higher concentrations of compounds had been applied. No coloration visible to the naked eye was observed at the highest doses (2500 µg).

Both peptides thus displayed anti-allergic activity that is dependent on the dose administered. The comparative peptide proved to be capable of inhibiting the inflammatory reaction completely at a dose of 25 µg (corresponding to 11.6 nmoles). The peptide of formula IB was able to inhibit the inflammatory reaction completely at a dose of 2.5 µg (approx. 0.8 nmoles), showing activity approx. 15 times greater than the activity of the comparative peptide. The full results, expressed as percentage inhibition of the allergic skin reaction (wheal), are shown in the following Table 2.

**TABLE 2**

| Dose of Peptide (µg/ml) | Peptide IB Inhibition of skin reaction (%) | Comparative peptide Inhibition of skin reaction (%) |
|---|---|---|
| 0.025 | 35 | 35 |
| 0.25 | 60 | 50 |
| 2.5 | 100 | 65 |
| 25 | 100 | 100 |
| 250 | 100 | 100 |
| 2500 | 100 | 100 |

Results similar to the results obtained with peptide IB were obtained with the following peptide:

IF (Ac-L-Cys(p-MeOBzl)-L-Arg-L-Thr-L-Tyr)₄-L-Lys₂-L-Lys-L-Lys-OH

### EXAMPLE 5

### Determination "in vivo" of the anti-allerqic activity of the peptide of formula (IB) and of the peptide of formula (H-D-Arg-D-Thr-D-Tyr)₄-L-Lys₂-L-Lys-Gly-OH (comparative peptide) using passive cutaneous anaphylaxis on the mouse ear as the experimental animal model.

Cutaneous passive anaphylaxis on the mouse ear was induced according to the procedure described by Inagaki et al., Int. Arch. Allergy Appl. Immunol. 1985, 78:113-117.

Rat ascitic fluid containing specific IgEs against the ovalbumin antigen was injected in the ear of female mice of the Balb/c strain. Ascitic fluid at different dilutions (5-10-20 times in PBS) was applied by intradermal injections at a volume of 10 µl, permitting the IgEs present in the ascitic fluid to bind to the FcεRs of the local mast cells, sensitizing them.

After 48 hours, 0.25 mg of ovalbumin and Evans Blue dye at a concentration of 1.8%, dissolved in 70 µl of PBS, were injected intravenously.

After 30 minutes, the appearance, on the mouse ear, of stained areas in the dermis, with extent and intensity of staining proportional to the quantity of antigen-specific IgEs injected with the ascitic fluid, was observed. The experiment included a negative control, represented by the intradermal injection of 10 µl of PBS without ascitic fluid, and there was no corresponding appearance of staining. The mice were sacrificed and the ears were removed for extraction and measurement of the dye discharged in the skin reaction. Each ear was dissolved in 0.5 ml of 1 M KOH, incubating at 37°C for 16 hours. After incubation, 2 ml of a mixture of 0.6M phosphoric acid and acetone in the ratio 5:13 (v:v) was added. The sample was then stirred and filtered, and finally the amount of dye was determined with a spectrophotometer, taking readings at 620 nm.

On the basis of the results obtained in the IgE-FcER linear binding assay, selecting as reference point the value of dilution of the ascitic fluid at which the most intense inflammatory reaction was observed, an assay of inhibition of the allergic reaction was performed using peptide IB and the comparative peptide.

As described previously, intradermal injections of 10 µl of ascitic fluid, diluted 20 times in PBS, were applied to the ear lobe of a group of Balb/c mice, permitting the IgEs present in the ascitic fluid to bind to the FcεRs of the local mast cells, sensitizing them.

After two days, various amounts of the peptides under investigation were dissolved in 100 µl of PBS and administered intraperitoneally to the sensitized mice. In particular, doses corresponding to 0.5 - 1 - 5 - 10 - 50 mg of peptide per kg of body weight of the mouse were used. 100 µl of PBS was injected as negative control. After 30 minutes the mice received the intravenous injection of the solution of ovalbumin and Evans Blue dye.

The results presented in Table 3, expressed as percentage inhibition of the allergic skin reaction, showed that both peptides displayed dose-dependent activity.

The comparative peptide produced 50% inhibition of the local allergic reaction at a dose of about 7 mg/kg, whereas the peptide of formula IB produced comparable inhibition at a dose of 1 mg/kg, thus displaying activity about 7 times greater than the activity of the comparative peptide. The full results, expressed as percentage inhibition of the allergic skin reaction, are shown in the following Table 3.

**TABLE 3**

| Dose of Peptide (mg/kg) | Peptide IB Inhibition of skin reaction (%) | Comparative peptide Inhibition of skin reaction (%) |
|---|---|---|
| 0.5 | 10 | 0 |
| 1.0 | 50 | 20 |
| 5.0 | 65 | 45 |
| 10.0 | 71 | 55 |
| 50.0 | 85 | 75 |

Results similar to the results obtained with peptide IB were obtained with the following peptide:

IF (Ac-L-Cys(p-MeOBzl)-L-Arg-L-Thr-L-Tyr)₄-L-Lys₂-L-Lys-L-Lys-OH

### EXAMPLE 6

### Determination "in vivo" of the anti-allergic activity of the peptide of formula (IB) and of the peptide of formula (H-D-Arq-D-Thr-D-Tyr)₄-L-Lys₂-L-Lys-Gly-OH (comparative peptide) using active cutaneous anaphylaxis on the mouse ear as the experimental animal model.

Active cutaneous anaphylaxis on the mouse ear was induced according to the procedure described by Inagaki et al., Japan. J. Pharmacol. 1992, 59:201-208.

Female mice of the Balb/c strain were immunized with the ovalbumin antigen in the presence of the adjuvant aluminium hydroxide gel (alum). The immunizations were carried out by intraperitoneal administration of 25 µg of ovalbumin and 1 mg of alum dissolved in 200 µl of PBS. A control group received intraperitoneal injections that only contained 1 mg of alum dissolved in 200 µl of PBS.

After two weeks, active cutaneous anaphylaxis was produced in the immunized animals by injecting different doses of ovalbumin (0.01 - 0.1 - 1 µg in 10 µl of PBS) in the dermis of the ear, and directly afterwards injecting the Evans Blue dye (1.8% w/v in a volume of 70 µl) intravenously, permitting the antigen to bind to the specific IgEs already present on the local mast cells, and thus inducing the local inflammatory reaction.

After 30 minutes, the appearance, on the mouse ear, of stained areas in the dermis, with extent and intensity of staining proportional to the quantity of allergen injected, was observed. The experiment included a negative control, represented by the intradermal injection of 10 µl of PBS without ovalbumin, and there was no corresponding appearance of staining. The mice were sacrificed and the ears were removed for extraction and measurement of the dye discharged in the skin reaction. Each ear was dissolved in 0.5 ml of 1 M KOH, incubating at 37°C for 16 hours. After incubation, 2 ml of a mixture of 0.6M phosphoric acid and acetone in the ratio 5:13 (v:v) was added. The sample was then stirred and filtered, and finally the amount of dye was determined with a spectrophotometer, taking readings at 620 nm.

On the basis of the results obtained in the IgE-FcεR linear binding assay, selecting as reference point the value of concentration of allergen at which the greatest allergic reaction was observed, an assay of inhibition of the allergic reaction was performed using peptide IB, with doses of 10 and 100 mg/kg, and the comparative peptide, with doses of 5, 10, 50 and 100 mg/kg.

The peptides were administered intraperitoneally to a group of Balb/c mice and, after thirty minutes, an intradermal injection of 0.1 µg of ovalbumin in PBS was effected in the ear lobe, followed by intravenous injection of the solution of dye. Extraction and quantification of the Evans Blue dye, discharged in the local inflammatory reaction, were carried out as described previously.

The results presented in Table 4, expressed as percentage inhibition of the allergic skin reaction, showed that both peptides administered intraperitoneally 30 minutes before inducing the inflammatory reactions, displayed dose-dependent activity in reduction of local allergic reactions.

In particular, the comparative peptide produced inhibition greater than 85% of the local allergic reaction at a dose equal to 100 mg/kg, whereas the peptide of formula IB produced comparable inhibition at a dose of 10 mg/kg, thus displaying activity approx. 15 times greater than the activity of the comparative peptide. The full results, expressed as percentage inhibition of the allergic skin reaction, are shown in the following Table 4.

**TABLE 4**

| Dose of Peptide (mg/kg) | Peptide IB Inhibition of skin reaction (%) | Comparative peptide Inhibition of skin reaction (%) |
|---|---|---|
| 5 | ND | 14 |
| 10 | 86 | 30 |
| 50 | ND | 75 |
| 100 | 100 | 87 |

Results similar to the results obtained with peptide IB were obtained with the following peptide:

IF (Ac-L-Cys(p-MeOBzl)-L-Arg-L-Thr-L-Tyr)₄-L-Lys₂-L-Lys-L-Lys-OH

## Claims

1. Peptide comprising the sequence of formula:
X₁-Arg-Thr-Tyr- (S)
where:
X₁ is the residue of an amino acid, of an amino acid derivative, of a carboxylic acid, or of some other derivative capable of binding covalently to the amino group of arginine, comprising at least one aromatic group.

2. Peptide of formula:
[X₁-Arg-Thr-Tyr]n-[R]m-[X₂]p
where:
X₁ is the residue of an amino acid, of a derivative of an amino acid, of a carboxylic acid, or of some other derivative capable of binding covalently to the amino group of arginine, said residue comprising at least one aromatic group,
X₂ is an amino acid, or a polypeptide sequence comprising from 2 to 8 amino acids,
R is an n-valent residue comprising one, two or three amino acids,
mis 0 or 1,
n is an integer from 1 to 4, and
p is 0 or 1.

3. Peptide according to Claim 1 or 2, **characterized in that** X₁ is selected from the group comprising the following amino acid residues: H-L-Tyr, H-D-Tyr, H-L-Phe, H-D-Phe, H-L-Trp, H-D-Trp, H-L-Phe, H-D-Phe, Ac-L-Tyr, Ac-D-Tyr, Ac-L-Phe, Ac-D-Phe, Ac-L-Trp, Ac-D-Trp, Ac-L-Phe, Ac-D-Phe.

4. Peptide according to Claim 1 or 2, **characterized in that** X₁ is selected from the group comprising the following residues of amino acid derivatives: H-L-Cys(p-MeOBzl), H-L-Cys(p-MeBzl), H-L-Cys(Bzl), H-D-Cys(p-MeOBzl), H-D-Cys(p-Me-Bzl), H-D-Cys(Bzl), Ac-L-Cys(p-MeOBzl), Ac-L-Cys(p-Me-Bzl), Ac-L-Cys(Bzl), Ac-D-Cys(p-MeOBzl), Ac-D-Cys(p-Me-Bzl), Ac-D-Cys(Bzl), H-L-Arg(Mts), H-L-Arg(Tos), H-D-Arg(Mts), H-D-Arg(Tos), Ac-L-Arg(Mts), Ac-L-Arg(Tos), Ac-D-Arg(Mts), Ac-D-Arg(Tos), H-L-Trp(CHO), H-D-Trp(CHO), Ac-L-Trp(CHO), Ac-D-Trp(CHO), H-L-Tyr(Bzl), H-L-Tyr(2Cl-Bzl), H-D-Tyr(Bzl), H-D-Tyr(2Cl-Bzl), Ac-L-Tyr(Bzl), Ac-L-Tyr(2Cl-Bzl), Ac-D-Tyr(Bzl), Ac-D-Tyr(2Cl-Bzl), L-Ser(Bzl), D-Ser(Bzl), L-Thr(Bzl), D-Thr(Bzl), L-Asp(Bzl), D-Asp(Bzl), L-Glu(Bzl), D-Glu(Bzl), Ac-L-Ser(Bzl), Ac-D-Ser(Bzl), Ac-L-Thr(Bzl), Ac-D-Thr(Bzl), Ac-L-Asp(Bzl), Ac-D-Asp(Bzl), Ac-L-Glu(Bzl), Ac-D-Glu(Bzl).

5. Peptide according to Claim 1 or 2, **characterized in that** X₁ is selected from the group comprising the following carboxylic acid residues: naphthylacetic, benzoic, 4-methoxybenzoic, 2,4-dichlorobenzoic, 2-nitrobenzoic, 4-chlorobenzoic, phenylacetic, diphenylacetic, (R)-2-phenylpropanoic, (S)-2-phenylpropanoic, (S)-3-phenylpropanoic, (R)-3-phenylpropanoic, (R,S)-2-methyl-3-phenylbutanoic, (S,R)-2-methyl-3-phenylbutanoic, (R,R)-2-methyl-3-phenylbutanoic, (S,S)-2-methyl-3-phenylbutanoic, (R,S)-2,3-diphenylpentanoic, (R,R)-2,3-diphenylpentanoic, (S,S)-2,3-diphenylpentanoic, (S,R)-2,3-dlphenylpentanoic, mono-(9H-fluoren-9-yl-methyl)carbonic acid, monobenzylcarbonic acid, mono-(2-chlorobenzyl)carbonic acid.

6. The peptide according to Claim 2, **characterized in that** X₂ is an amino acid selected from the group comprising Lys, Orn, and Dap, or a polypeptide sequence comprising from 2 to 8 amino acids represented by the formula:
(A)q-B
where q is an integer from 1 to 7,
A is Gly, Ala or, when q is at least 2, a polypeptide sequence comprising Gly and Ala, and
B is Lys, Orn, or Dap.

7. Peptide according to Claim 6, **characterized in that** X₂ is a polypeptide sequence selected from the group comprising: Gly-Lys, Gly-Orn, Gly-Dap, Ala-Lys, Ala-Orn, Ala-Dap, Gly-Gly-Lys, Gly-Gly-Orn, Gly-Gly-Dap, Gly-Ala-Lys, Gly-Ala-Orn, Gly-Ala-Dap, Ala-Gly-Lys, Ala-Gly-Orn, Ala-Gly-Dap, Ala-Ala-Lys, Ala-Ala-Orn, Ala-Ala-Dap, Gly-Gly-Gly-Lys, Gly-Gly-Gly-Orn, Gly-Gly-Gly-Dap, Ala-Ala-Ala-Lys, Ala-Ala-Ala-Orn, Ala-Ala-Ala-Dap, Gly-Gly-Gly-Gly-Lys, Gly-Gly-Gly-Gly-Orn, Gly-Gly-Gly-Gly-Dap, Gly-Gly-Gly-Gly-Gly-Lys, Gly-Gly-Gly-Gly-Gly-Orn, Gly-Gly-Gly-Gly-Gly-Dap, Gly-Ala-Gly-Ala-Gly-Lys, Gly-Ala-Gly-Ala-Gly- Orn, Gly-Ala-Gly-Ala-Gly-Dap, Ala-Gly-Ala-Gly-Ala-Gly-Ala-Lys, Ala-Gly-Ala-Gly-Ala-Gly-Ala-Orn, Ala-Gly-Ala-Gly-Ala-Gly-Ala-Dap.

8. Peptide according to Claim 2, **characterized in that** n is equal to two.

9. Peptide according to Claim 8, **characterized in that** R is selected from the group comprising lysine (Lys), ornithine (Orn), diaminopropionic acid (Dap) and diaminobutyric acid (Dab).

10. Peptide according to Claim 2, **characterized in that** n is equal to three.

11. Peptide according to Claim 10, **characterized in that** R is selected from the group comprising a dipeptide of formula (Lys-Lys), (Orn-Orn), (Dap-Dap) and (Dab-Dab).

12. Peptide according to Claim 2, **characterized in that** n is equal to four.

13. Peptide according to Claim 12, **characterized in that** R is selected from the group comprising a branched tripeptide of formula Lys(εLys)-Lys, Orn(δOrn)-Orn, Dap(βDap)-Dap, and Dab(yDab)-Dab, where the residue in parentheses is bound to the amine function in position ε, δ, β or γ, respectively, of the residue that precedes it.

14. Peptide according to Claim 2, **characterized in that** R comprises the tripeptide Lys(εLys)-Lys, X₁ is selected from the group comprising an acetylated amino acid and a carboxylic acid, X₂ comprises L-Lys, and the amino acids of the tripeptide Arg-Thr-Tyr are in configuration D.

15. Peptide according to Claim 1 or 2, **characterized in that** it is selected from the group comprising:
IA (H-D-Cys(p-MeOBzI)-D-Arg-D-Thr-D-Tyr)₄-L-Lys₂-L-Lys-L-Lys-OH
IB (H-L-Cys(p-MeOBzI)-D-Arg-D-Thr-D-Tyr)₄-L-Lys₂-L-Lys-L-Lys-OH
IC (H-D-Cys(p-MeOBzI)-L-Arg-L-Thr-L-Tyr)₄-L-Lys₂-L-Lys-L-Lys-OH
ID (H-L-Cys(p-MeOBzI)-L-Arg-L-Thr-L-Tyr)₂-Lys-Gly-Lys-OH
IE H-L-Cys(p-MeOBzI)-D-Arg-D-Thr-D-Tyr-Gly-L-Ala-Gly-L-Ala-Gly-L-Orn-OH
IF (Ac-L-Cys(p-MeOBzl)-L-Arg-L-Thr-L-Tyr)₄-L-Lys₂-L-Lys-L-Lys-OH
IG H-L-Cys(p-MeOBzl)-D-Arg-D-Thr-D-Tyr-Gly-L-Ala-Gly-L-Ala-Gly-L-Lys-OH
IH (H-L-Cys(p-MeOBzl)-L-Arg-L-Thr-L-Tyr)₂-Lys-Lys*(CO-L-Tyr-L-Thr-L-Arg-L-Cys(p-MeOBzl))-OH
IL (H-L-Cys(p-MeOBzl)-L-Arg-L-Thr-L-Tyr)₄-L-Orn₂-L-Orn-L-Orn-OH
IM ( H-L-Cys(p-MeOBzl)-L-Arg-L-Thr-L-Tyr)₂-Orn-Gly-OH
IN (Ac-L-Cys(p-MeOBzI)-L-Arg-L-Thr-L-Tyr)₄-L-Orn₂-L-Orn-L-Orn-OH
IO (H-L-Cys(p-MeOBzI)-L-Arg-L-Thr-L-Tyr)₂-L-Orn-L-Orn*(CO-L-Tyr-L-Thr-L-Arg-L-Cys(p-MeOBzl))-OH
IP (Ac-L-Cys(p-MeOBzI)-D-Arg-D-Thr-D-Tyr)₄-L-Lys₂-L-Lys-L-Lys-OH
IQ (Ac-D-Cys(p-MeOBzI)-L-Arg-L-Thr-L-Tyr)₄-L-Lys₂-L-Lys-L-Lys-OH
IR (Ac-L-Cys(p-MeOBzI)-L-Arg-L-Thr-L-Tyr)₄-L-Lys₂-L-Lys-L-Lys-NH₂
IS (Ac-D-Cys(p-MeOBzI)-D-Arg-L-Thr-D-Tyr)₄-L-Lys₂-L-Lys-L-Lys-NH₂
IT (Ac-D-Cys(p-MeOBzI)-L-Arg-D-Thr-L-Tyr)₄-L-Lys₂-L-Lys-L-Lys-OH
IU (Ac-L-Cys(p-MeOBzI)-D-Arg-D-Thr-L-Tyr)₄-L-Lys₂-L-Lys-L-Lys-OH

16. Pharmaceutical composition comprising a biologically effective dose of a peptide according to any one of Claims 1 to 15 and at least one pharmaceutically acceptable excipient.

17. Use of a peptide according to any one of Claims 1 to 15 for the manufacture of a pharmaceutical composition for the treatment of allergic diseases.
